# EUROPEAN PATENT SPECIFICATION

(11) **EP 1 535 613 B1**
(45) Date of publication and mention of the grant of the patent: **25.08.2010**
(21) Application number: 04027375.7
(22) Date of filing: 16.11.2000
(51) Int. Cl.: A61K 31/40, C07D 207/335

(54) **Process for preparing a polymorphic form of atorvastatin calcium**
Verfahren zur Herstellung einer polymorphen Form von Atorvastatin calcium
Procédé pour la préparation d'une form polymorphe de atorvastatin calcium

(30) Priority: 17.11.1999 US 166153 P
(43) Date of publication of application: 01.06.2005
(62) Divisional of application: 00978744.1
(73) Proprietor: TEVA PHARMACEUTICAL INDUSTRIES, LTD., 49131 Petah Tiqva (IL)
(72) Inventor: Ayalon, Ari, Ramat Golda,Haifa 34982 (IL); Levinger, Michal, Nofit (IL); Roytblat, Sofia, Haifa (IL); Niddam, Valerie, Ein Vered 40696 (IL); Lifshitz, Revital, 46322 Herzilia (IL); Aronhime, Judith, 76217 Rehovot (IL)
(74) Representative: Gallagher, Kirk James

(56) References cited:
- EP-A- 0 247 633
- EP-A- 0 409 281
- WO-A-94/16693
- WO-A-97/03958
- US-A- 5 273 995
- US-A- 5 969 156

## Description

### FIELD OF THE INVENTION

The present invention relates to processes for preparing a novel crystalline form of atorvastatin calcium.

### BACKGROUND OF THE INVENTION

Atorvastatin is a member of the class of drugs called statins. Statin drugs are currently the most therapeutically effective drugs available for reducing low density lipoprotein (LDL) particle concentration in the blood stream of patients at risk for cardiovascular disease. A high level of LDL in the bloodstream has been linked to the formation of coronary lesions which obstruct the flow of blood and can rupture and promote thrombosis. Goodman and Gilman, *The Pharmacological Basis of Therapeutics* 879 (9th ed. 1996). Reducing plasma LDL levels has been shown to reduce the risk of clinical events in patients with cardiovascular disease and patients who are free of cardiovascular disease but who have hypercholesterolemia. Scandinavian Simvastatin Survival Study Group, 1994; Lipid Research Clinics Program, 1984a, 1984b.

The mechanism of action of statin drugs has been elucidated in some detail. They interfere with the synthesis of cholesterol and other sterols in the liver by competitively inhibiting the 3-hydroxy-3-methyl-glutaryl-coenzyme A reductase enzyme ("HMG-CoA reductase"). HMG-CoA reductase catalyzes the conversion HMG to mevalonate, which is the rate determining step in the biosynthesis of cholesterol, and so, its inhibition leads to a reduction in the concentration of cholesterol in the liver. Very low density lipoprotein (VLDL) is the biological vehicle for transporting cholesterol and triglycerides from the liver to peripheral cells. VLDL is catabolized in the peripheral cells which releases fatty acids which may be stored in adipocytes or oxidized by muscle. The VLDL is converted to intermediate density lipoprotein (IDL), which is either removed by an LDL receptor, or is converted to LDL. Decreased production of cholesterol leads to an increase in the number of LDL receptors and corresponding reduction in the production of LDL particles by metabolism of IDL.

Atorvastatin is the common chemical name of [*R*-(*R**,*R**)]-2-(4-fluorophenyl)-β,δ-dihydroxy-5-(1-methylethyl)-3-phenyl-4-[(phenylamino)carbonyl]-1H-pyrrole-1-heptanoic acid. The free acid is prone to lactonization. The molecular structure of the lactone is represented by formula (I).

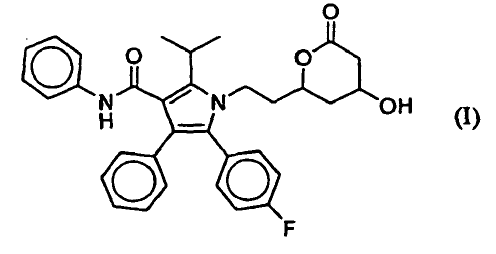

Atorvastatin is marketed as the hemi calcium salt-trihydrate under the name LIPITOR by Warner-Lambert Co.

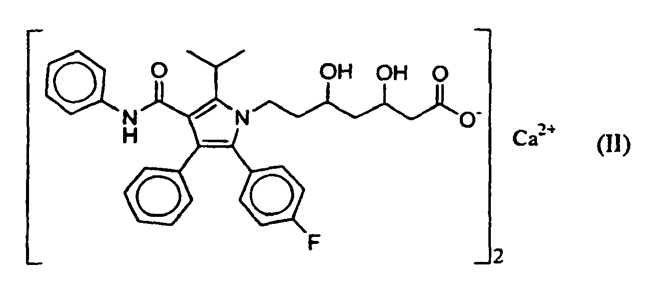

Atorvastatin was first disclosed to the public and claimed in U.S. Patent No. 4,681,893. The hemi calcium salt depicted in formula (II) (hereafter "atorvastatin calcium") is disclosed in U.S. Patent No. 5,273,995. This patent teaches that the calcium salt is obtained by crystallization from a brine solution resulting from the transposition of the sodium salt with CaCl₂ and further purified by recrystallization from a 5:3 mixture of ethyl acetate and hexane. Both of these U.S. patents are hereby incorporated by reference.

The present invention includes a process for preparing a new crystal form of atorvastatin calcium in both hydrate and anhydrate states. Polymorphism is the property of some molecules and molecular complexes to assume more than one crystalline or amorphous form in the solid state. A single molecule, like the atorvastatin in formula (I) or the salt complex of formula (II), may give rise to a variety of solids having distinct physical properties like solubility, X-ray diffraction pattern and solid state ¹³C NMR spectrum. The differences in the physical properties of polymorphs result from the orientation and intermolecular interactions of adjacent molecules (complexes) in the bulk solid. Accordingly, polymorphs are distinct solids sharing the same molecular formula, which may be thought of as analogous to a unit cell in metallurgy, yet having distinct advantageous and/or disadvantageous physical properties compared to other forms in the polymorph family. One of the most important physical properties of pharmaceutical polymorphs is their solubility in aqueous solution, particularly their solubility in the gastric juices of a patient. For example, where absorption through the gastrointestinal tract is slow, it is often desirable for a drug that is unstable to conditions in the patient's stomach or intestine to dissolve slowly so that it does not accumulate in a deleterious environment. On the other hand, where the effectiveness of a drug correlates with peak bloodstream levels of the drug, a property shared by statin drugs, and provided the drug is rapidly absorbed by the GI system, then a more rapidly dissolving form is likely to exhibit increased effectiveness over a comparable amount of a more slowly dissolving form.

U.S. Patent No. 5,969,156 discloses three polymorphs of atorvastatin designated Forms I, II, and IV by the inventors of those forms. While the inventors of U.S. Patent No. 5,969,156 claim certain processing and therapeutic advantages of their forms over amorphous atorvastatin calcium, advantages may yet be realized by other heretofore undiscovered forms of atorvastatin calcium.

### SUMMARY OF THE INVENTION

The present invention provides a process for preparing a new Form V of atorvastatin calcium in both anhydrate and hydrate states, which possesses the advantage of higher solubility in water than atorvastatin Form I.

### BRIEF DESCRIPTION OF THE DRAWINGS

FIG. **1** is an X-ray powder diffractogram of atorvastatin calcium Form V.
FIG. **2** is a solid state ¹³C NMR spectrum of atorvastatin calcium Form V.

### DETAILED DESCRIPTION OF THE INVENTION

The new crystalline form of atorvastatin calcium Form V is well distinguished from the crystal modifications obtained by carrying out the procedures described in U.S. Patents Nos. 5,273,995 and 5,969,156 using X-ray powder diffraction and solid-state ¹³C nuclear magnetic resonance techniques.

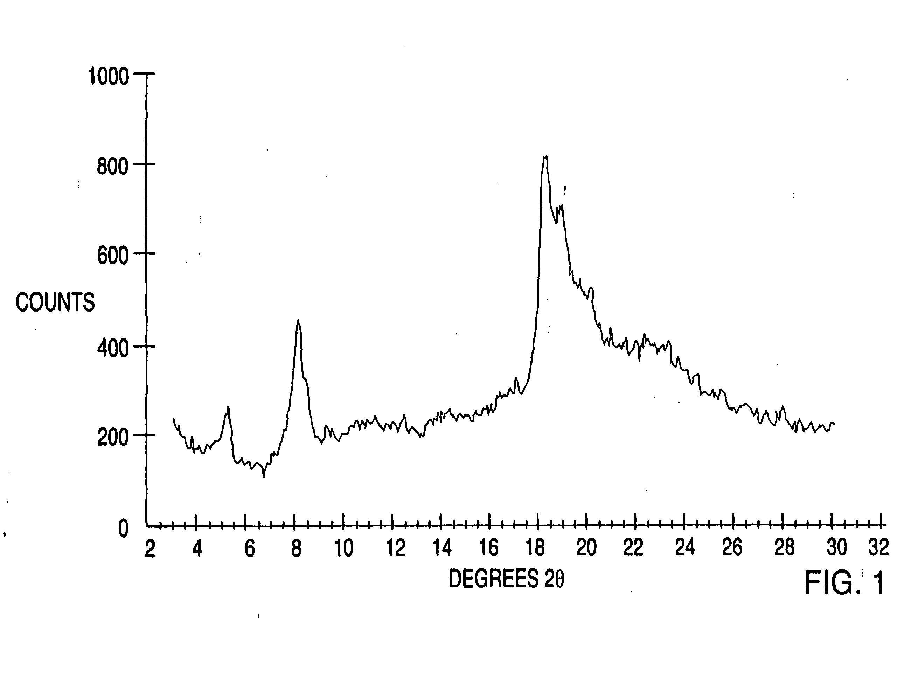

The X-ray powder diffractogram of Form V (Fig.1) has two medium peaks at 5.3±0.2 and 8.3±0.2 degrees 2θ and one large peak in the range 18-23 degrees 26 with a maximum at about 18.3±0.2 degrees two-theta. This X-Ray pattern is well distinguished from that of known Forms I, II, III and IV and also is well distinguished from the X-Ray pattern of amorphous atorvastatin calcium which is characterized by two broad humps in the ranges 8-14 degrees 2θ and 15-26 degrees 2θ. The X-ray powder diffractogram of Fig. 1 was obtained by methods known in the art using a Philips X-ray powder diffractometer with a curved graphite monochromator using goniometer model 1050/70. Copper radiation of λ = 1.5418 Å was used. Measurement range: 3-30 degrees 2θ.

The solid-state ¹³C NMR spectrum of Form V is characterized by the following chemical shifts:

| δ (ppm) | |
|---|---|
| 21.9 | |
| 25.9 | |
| 40.4 | |
| 41.8 | |
| 42.3 | |
| 63-73 | (two broad peaks) |
| 115.6 | |
| 118.9 | |
| 122.5 | |
| 128.7 | (strong) |
| 135.1 | |
| 161.0 | |
| 167.1 | |
| 176-186 | (broad peak) |

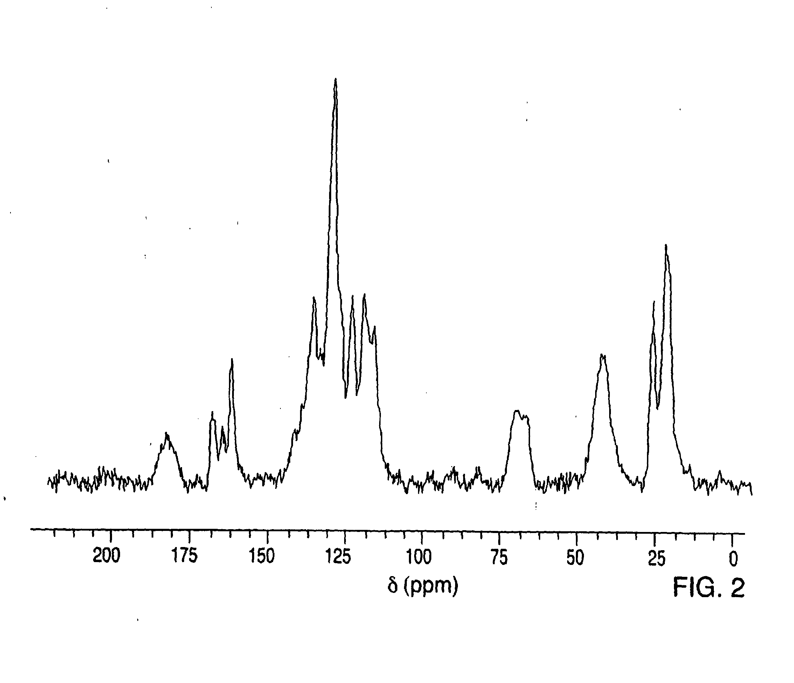

This solid-state ¹³C NMR spectrum (Fig. 2) is well distinguished from those of known Forms I, II, III and IV, and also is distinguished from that of the amorphous form which displays a different pattern with shifts significantly different from that of Form V at 21.0 ppm, 26.4 ppm, one broad peak in the range 60-75 ppm with a maximum at 69.7 ppm and 138.8 ppm. The spectrum of Fig. 2 was obtained on a Bruker DMX-500 digital F NMR spectrometer operating at 125.76 MHz. The instrument was equipped with a BL-4 cpmas probehead and a high resolution/high performance (HPHP) ¹H for solids. The magic angle and proton decoupling efficiency were optimized before acquisition. The sample was spun at 5.0 kHz spin rate on 4 mm zirconia rotors.

Atorvastatin calcium Form V may contain up to 12% water, which corresponds to the stoichiometric value of 9 water molecules per molecule of atorvastatin calcium. Thus, atorvastatin calcium Form V can be in various states of hydration, between 0 and 9 moles of water.

The present invention provides a process for the preparation of atorvastatin calcium Form V. The process comprises the steps of dissolving a salt of atorvastatin in a solvent to form an atorvastatin salt solution, optionally removing impurities from the atorvastatin salt solution, contacting the atorvastatin salt solution with a calcium salt and isolating atorvastatin calcium in new Form V.

The atorvastatin salt of the present invention includes alkali metal salts, e.g. lithium, sodium, and potassium salts; alkaline-earth metal salts such as magnesium salts; as well as ammonium and alkyl, aryl or alkaryl ammonium salts. The preferred atorvastatin salts are alkali metal salts; most preferred is the sodium salt.

Any solvent capable of dissolving the atorvastatin salt and from which atorvastatin calcium Form V may be isolated is a suitable solvent of the invention. The choice of solvent will therefore depend upon the selection of the atorvastatin salt and the calcium salt. The solvent should be selected from those in which the atorvastatin salt and calcium salt are at least sparingly soluble. By sparingly soluble is meant not substantially less soluble than 0.02 g/ml at 50-60°C for the atorvastatin salt and not substantially less soluble than 0.0002 M at 10-15 °C for the calcium salt.

Suitable solvents include but are not limited to hydroxylic solvents like water, alcohols and mixtures thereof, including hydroxylic solvents and hydroxylic solvent mixtures which have been made either acidic or basic by addition of a mineral acid or base. Preferred solvents are water, methanol. ethanol and mixtures thereof.

The calcium salt of the present invention includes organic and inorganic salts of calcium which are capable of dissociating into Ca²⁺ and an anionic component when added to the atorvastatin salt solution. Among the organic salts that may be used are carboxylates and sulfonates. Among the carboxylates are lower alkyl carboxylates like acetate, proprionate, butyrate and tartrate and aryl carboxylates like benzoate and phthalate as well as higher alkyl carboxylates like stearate, dodecanoate and the like. Also included are calcium ascorbate and succinate. Among the sulfonates that may be used are lower alkyl and aryl sulfonates like calcium methane sulfonate, calcium benzene sulfonate and calcium *p*-toluene sulfonate. The preferred organic calcium salts are lower carboxylate salts, the most preferred organic calcium salt is calcium acetate.

Depending upon solubility, inorganic salts which may be used include halide salts such as CaCl₂, CaF₂, CaBr₂ and CaI₂, as well as calcium borate (B₄CaO₇), calcium tetrafluoroborate (CaBF₄), calcium carbonate (CaCO₃), monobasic calcium phosphate (Ca(H₂PO₄)₂), dibasic calcium phosphate (CaHPO₄) and tribasic calcium phosphate (Ca(PO₄)₂), calcium sulfate (CaSO₄) and calcium hydroxide (Ca(OH)₂), and hydrates thereof.

Whether organic or inorganic, the calcium salt is preferably added in an amount that provides one half mole of Ca²⁺ per mole of atorvastatin in the atorvastatin salt solution. For example, if the atorvastatin salt is atorvastatin sodium (atorvastatin⁻ Na⁺), then about one half mole of calcium salt per mole of the atorvastatin salt is appropriate. If the atorvastatin salt is atorvastatin magnesium ([atorvastatin⁻]₂ Mg²⁺), then about one mole of calcium salt per mole of atorvastatin salt is appropriate. Otherwise, mixed salts containing atorvastatin may form.

The calcium salt may be contacted with the atorvastatin salt solution by adding the calcium salt in substantially pure form, *i*.*e*. either as a solid or, if liquid, as a neat liquid, to the atorvastatin salt solution or, preferably, by first forming a calcium salt solution and then contacting the atorvastatin salt solution and calcium salt solution. It is most preferred to contact the calcium salt and the atorvastatin salt solution by first dissolving the calcium salt in a solvent and then adding the calcium salt solution to the atorvastatin salt solution slowly. Suitable calcium salt solvents are solvents previously mentioned as being suitable solvents for the atorvastatin salt, provided the calcium salt is at least sparingly soluble in the particular solvent.

In a particularly preferred embodiment, wherein the atorvastatin salt is an atorvastatin alkali metal salt and the atorvastatin salt solvent is a 1:2 methanol:water mixture, the preferred calcium salt is calcium acetate and the preferred calcium salt solvent is water. When the calcium salt solvent is water, it is preferably used in an amount that provides about a 20 to 30 millimolar solution of the calcium salt, more preferably about a 25 millimolar solution.

In addition, the atorvastatin and calcium salts are preferably combined at elevated temperature and at concentrations disclosed above and in the examples, which follow, in order that crystallization of Form V may be induced by cooling of the so-formed atorvastatin calcium solution. The elevated temperature is preferably above 40°C and below 80°C, more preferably above 50°C and below 70°C and most preferably about 60°C. One skilled in the art will appreciate that by adjusting temperature and concentration, the yield of atorvastatin calcium Form V may be optimized. Crystallization of atorvastatin calcium Form V may also be induced by addition of a seed crystal of atorvastatin calcium, preferably Form V although other forms also may be used.

Once crystals of atorvastatin Form V have crystallized, either spontaneously, upon cooling, upon seeding or by another inducement, the crystals may be isolated by filtration or other conventional means known to the art. The isolated crystals may also be dried by conventional means.

It has also been found that atorvastatin calcium can be crystallized in Form V by dissolving atorvastatin calcium in THF or alcohols like methanol or ethanol, and subsequently adding water as an antisolvent.

Having thus described the various aspects of the present invention, the following examples are provided to illustrate specific embodiments of the present invention. They are not intended to be limiting in any way.

### EXAMPLES

### EXAMPLE 1

The sodium salt of atorvastatin (52.2 g) was dissolved in methanol (510 ml) and then diluted with water (1 L). The resulting solution was transferred to a separatory funnel containing 1:1 ethyl acetate/hexane (1 L). The phases were mixed by bubbling nitrogen gas through the separatory funnel. Upon cessation of nitrogen flow, the phases separated and the upper, organic, phase was removed. The lower, aqueous, phase was washed with 1:1 ethyl acetate/hexane (1 L) and then transferred to a round bottom flask. Active charcoal (10.2 g) was added. The flask was heated to 50 °C and the solution was stirred for two hours. The activated charcoal was then removed by filtration through celite, the charcoal and celite being rinsed with methanol (1540 ml), and the rinsate and filtrate then being combined into one atorvastatin sodium salt solution.

The quantity of atorvastatin obtained by purification was determined by calibrated HPLC analysis of the purified atorvastatin sodium salt solution. Based on this analysis, a quantity of calcium acetate (8.38 g, 0.5 eq.) was dissolved in water (1.9 L) and heated to 60 °C. The atorvastatin sodium salt solution was heated to 63 °C and the solutions were combined by slow addition of the calcium acetate solution to the atorvastatin sodium salt solution. Upon completing the addition, the mixture was cooled. Crystallization of Form V began to occur at a temperature of 43 °C and cooling was continued until the flask temperature reached 13 °C.

The crystals were isolated by slow vacuum filtration and then dried over anhydrous silica for 5 days to yield atorvastatin calcium salt Form V.

### EXAMPLE 2

Atorvastatin calcium (10 g) was dissolved in methanol (400 ml) at room temperature. Water (300 ml) was added slowly to the methanolic solution with stirring and the resulting solution was heated to 60°C. The solution was then cooled to between 10 and 15 °C within 3 h. Precipitation started at about 40°C. The thick slurry was then dried at 50°C under reduced pressure for 48 h to yield atorvastatin calcium Form V.

### EXAMPLE 3

Atorvastatin calcium (5 g) was dissolved in methanol (100 ml) at room temperature. To this methanolic solution, water (100 ml) was added while stirring. Precipitation occurred instantly and after cooling the slurry to 15°C the precipitate was filtered and dried at 50°C under reduced pressure for 48 h to yield atorvastatin calcium Form V.

### EXAMPLE 4

Atorvastatin calcium (5 g) was dissolved in methanol (200 ml). The methanolic solution was placed into a stirred reactor containing water (150 ml) at 45°C. The obtained slurry was cooled to 10°C, filtered and dried at 50°C under reduced pressure for 48 h to yield atorvastatin calcium Form V.

### EXAMPLE 5

Atorvastatin calcium (1 g) was dissolved in ethanol (15 ml) after heating. To this ethanolic solution, water (10 ml) was added while stirring. Precipitation occurred instantly. The gel-like precipitate was filtered without vacuum and dried at 50°C under reduced pressure for 24 h to yield atorvastatin calcium Form V.

### EXAMPLE 6

Atorvastatin calcium (1 g) was dissolved in THF (25 ml) at room temperature. To this solution, water (60 ml) was added while stirring. The reaction mixture was stirred for 18 hours at room temperature and the precipitate (gel) was filtered without vacuum and dried at 50°C under reduced pressure for 24 h to yield atorvastatin calcium Form V.

The invention has been described with reference to its preferred embodiments. From this description, those skilled in the art may appreciate changes that could be made in the invention which do not depart from the scope and spirit of the invention as described above and claimed hereafter.

## Claims

1. A process for preparing atorvastatin calcium, or hydrate thereof, **characterised by** data selected from the group consisting of an X-ray diffraction pattern having peaks at 5.3 ± 0.2 and 8.3 ± 0.2 degrees two theta and a broad peak at 18-23 ± 0.2 degrees two theta; and a solid state ¹³C NMR spectrum having signals at 21.9, 25.9, 118.9, 122.5, 128.7, 161.0 and 167.1 ppm,
comprising the steps of
a) dissolving a metal, ammonium or alkylammonium salt of atorvastatin in a solvent to form an atorvastatin salt solution,
b) removing impurities from the atorvastatin salt solution,
c) contacting the atorvastatin salt solution with a calcium salt, and
d) isolating the atorvastatin calcium or hydrate thereof.

2. A process for preparing atorvastatin calcium, or hydrate thereof, **characterised by** data selected from the group consisting of an X-ray diffraction pattern having peaks at 5.3 ± 0.2 and 8.3 ± 0.2 degrees two theta and a broad peak at 18-23 ± 0.2 degrees two theta; and a solid state ¹³C NMR spectrum having signals at 21.9, 25.9, 118.9, 122.5, 128.7, 161.0 and 167.1 ppm, comprising the steps of
a) dissolving a metal, ammonium or alkylammonium salt of atorvastatin in a solvent to form an atorvastatin salt solution,
b) contacting the atorvastatin salt solution with a calcium salt,
c) crystallizing the atorvastatin calcium or hydrate thereof by addition of a seed crystal of a atorvastatin calcium, and
d) isolating the atorvastatin calcium or hydrate thereof.

3. The process of claim 2 wherein the seed crystal of atorvastatin calcium is atorvastatin calcium, or hydrate thereof, **characterised by** data selected from the group consisting of an X-ray diffraction pattern having peaks at 5.3 ± 0.2 and 8.3 ± 0.2 degrees two theta and a broad peak at 18-23 ± 0.2 degrees two theta; and a solid state ¹³C NMR spectrum having signals at 21.9, 25.9, 118.9, 122.5, 128.7, 161.0 and 167.1 ppm

4. The process of any of claims 1 to 3 wherein the calcium salt is added in an amount that provides one half mole of Ca²⁺ per mole of atorvastatin in the atorvastatin salt solution.

5. The process of claim 4 wherein the atorvastatin and calcium salts are combined at an elevated temperature.

6. The process of claim 5 wherein the elevated temperature is above 40°C and below 80°C.

7. The process of claim 6 wherein the elevated temperature is above 50°C and below 70°C.

8. The process of any preceding claim wherein the salt of atorvastatin is a metal salt of atorvastatin.

9. The process of claim 8 wherein the metal salt of atorvastatin is a sodium salt of atorvastatin.

10. The process of any preceding claim wherein the calcium salt is calcium acetate.

## Patentansprüche

1. Verfahren zur Herstellung von Atorvastatin-Kalzium oder einem Hydrat davon, welches durch Daten gekennzeichnet ist, ausgewählt aus der Gruppe, bestehend aus einem Röntgenbeugungsmuster mit Peaks bei 5,3 ± 0,2 und 8,3 ± 0,2 Grad Zwei-Theta und einem breiten Peak bei 18-23 ± 0,2 Grad Zwei-Theta und einem Festphasen ¹³C-NMR-Spektrum mit Signalen bei 21,9, 25,9, 118,9, 122,5, 128,7, 161,0 und 167,1 ppm, welches die folgenden Stufen umfasst:
a) Lösen eines Metall-, Ammonium- oder Alkylammoniumsalzes von Atorvastatin in einem Lösungsmittel, unter Bildung einer Atorvastatinsalzlösung,
b) Entfernen von Verunreinigungen aus der Atorvastatinsalzlösung,
c) In Kontakt bringen der Atorvastatinsalzlösung mit einem Kalziumsalz und
d) Isolieren von Atorvastatin-Kalzium oder dem Hydrat davon.

2. Verfahren zur Herstellung von Atorvastatin-Kalzium oder einem Hydrat davon, welches durch Daten gekennzeichnet ist, ausgewählt aus der Gruppe, bestehend aus einem Röntgenbeugungsmuster mit Peaks bei 5,3 ± 0,2 und 8,3 ± 0,2 Grad Zwei-Theta und einem breiten Peak bei 18-23 ± 0,2 Grad Zwei-Theta und einem Festphasen ¹³C-NMR-Spektrum mit Signalen bei 21,9, 25,9, 118,9, 122,5, 128,7, 161,0 und 167,1 ppm, welches die folgenden Stufen umfasst:
a) Lösen eines Metall-, Ammonium- oder Alkylammoniumsalzes von Atorvastatin in einem Lösungsmittel, unter Bildung einer Atorvastatinsalzlösung,
b) Inkontaktbringen der Atorvastatinsalzlösung mit einem Kalziumsalz,
c) Kristallisieren des Atorvastatin-Kalziums oder Hydrats davon durch Zugabe eines Keimkristalls aus einem Atorvastatin-Kalzium und
d) Isolieren von Atorvastatin-Kalzium oder dem Hydrat davon.

3. Verfahren gemäß Anspruch 2, wobei der Keimkristall von Atorvastatin-Kalzium ein Atorvastatin-Kalzium oder Hydrat davon ist, welches durch Daten gekennzeichnet ist, ausgewählt aus der Gruppe, bestehend aus einem Röntgenbeugungsmuster mit Peaks bei 5,3 ± 0,2 und 8,3 ± 0,2 Grad Zwei-Theta und einem breiten Peak bei 18-23 ± 0,2 Grad Zwei-Theta und einem Festphasen ¹³C-NMR-Spektrum mit Signalen bei 21,9, 25,9, 118,9, 122,5, 128,7, 161,0 und 167,1 ppm.

4. Verfahren gemäß einem der Ansprüche 1 bis 3, wobei das Kalziumsalz in einer Menge zugegeben wird, die ein halbes Mol Ca²⁺ pro Mol Atorvastatin in der Atorvastatinsalzlösung zur Verfügung stellt.

5. Verfahren gemäß Anspruch 4, wobei die Atorvastatin- und Kalziumsalze bei einer erhöhten Temperatur zusammengegeben werden.

6. Verfahren gemäß Anspruch 5, wobei die erhöhte Temperatur über 40 °C und unter 80 °C liegt.

7. Verfahren gemäß Anspruch 6, wobei die erhöhte Temperatur über 50 °C und unter 70 °C liegt.

8. Verfahren gemäß einem der vorangehenden Ansprüche, wobei das Salz von Atorvastatin ein Metallsalz von Atorvastatin ist.

9. Verfahren gemäß Anspruch 8, wobei das Metallsalz von Atorvastatin ein Natriumsalz von Atorvastatin ist.

10. Verfahren gemäß einem der vorangehenden Ansprüche, wobei das Kalziumsalz Kalziumacetat ist.

## Revendications

1. Procédé de préparation d'atorvastatine calcique ou d'un hydrate de celle-ci, **caractérisé par** des données choisies dans le groupe constitué d'un diagramme de diffraction des rayons X ayant des pics à 5,3 ± 0,2 et 8,3 ± 0,2 degrés deux thêta et un pic large à 18-23 ± 0,2 degrés deux thêta ; et un spectre ¹³C RMN à l'état solide ayant des signaux à 21,9, 25,9, 118,9, 122,5, 128,7, 161,0 et 167,1 ppm, comprenant les étapes consistant en
a) la dissolution d'un métal, d'un sel d'ammonium ou d'alkylammonium d'atorvastatine dans un solvant pour former une solution de sel d'atorvastatine,
b) le retrait des impuretés de la solution de sel d'atorvastatine,
c) la mise en contact de la solution de sel d'atorvastatine avec un sel de calcium, et
d) l'isolement de l'atorvastatine calcique ou d'un hydrate de celle-ci.

2. Procédé de préparation d'atorvastatine calcique ou d'un hydrate de celle-ci, **caractérisé par** des données choisies dans le groupe constitué d'un diagramme de diffraction des rayons X ayant des pics à 5,3 ± 0,2 et 8,3 ± 0,2 degrés deux thêta et un pic large à 18-23 ± 0,2 degrés deux thêta ; et un spectre ¹³C RMN à l'état solide ayant des signaux à 21,9, 25,9, 118,9, 122,5, 128,7, 161,0 et 167,1 ppm, comprenant les étapes consistant en
a) la dissolution d'un métal, d'un sel d'ammonium ou d'alkylammonium d'atorvastatine dans un solvant pour former une solution de sel d'atorvastatine,
b) la mise en contact de la solution de sel d'atorvastatine avec un sel de calcium,
c) la cristallisation de l'atorvastatine calcique ou d'un hydrate de celle-ci par addition d'un cristal d'ensemencement d'une atorvastatine calcique, et
d) l'isolement de l'atorvastatine calcique ou d'un hydrate de celle-ci.

3. Procédé selon la revendication 2, dans lequel le cristal d'ensemencement de l'atorvastatine calcique est l'atorvastatine calcique ou un hydrate de celle-ci, **caractérisé par** les données choisies dans le groupe constitué d'un diagramme de diffraction des rayons X ayant des pics à 5,3 ± 0,2 et 8,3 ± 0,2 degrés deux thêta et un pic large à 18-23 ± 0,2 degrés deux thêta ; et un spectre ¹³C RMN à l'état solide ayant des signaux à 21,9, 25,9, 118,9, 122,5, 128,7, 161,0 et 167,1 ppm.

4. Procédé selon l'une quelconque des revendications 1 à 3, dans lequel le sel de calcium est ajouté en une quantité qui donne une demi-mole de Ca²⁺ par mole d'atorvastatine dans la solution de sel d'atorvastatine.

5. Procédé selon la revendication 4, dans lequel l'atorvastatine et les sels de calcium sont combinés à une température élevée.

6. Procédé selon la revendication 5, dans lequel la température élevée est supérieure à 40° C et inférieure à 80° C.

7. Procédé selon la revendication 6, dans lequel la température élevée est supérieure à 50° C et inférieure à 70° C.

8. Procédé selon l'une quelconque des revendications précédentes, dans lequel le sel d'atorvastatine est un sel métallique d'atorvastatine.

9. Procédé selon la revendication 8, dans lequel le sel métallique d'atorvastatine est un sel de sodium d'atorvastatine.

10. Procédé selon l'une quelconque des revendications précédentes, dans lequel le sel de calcium est l'acétate de calcium.
